(19) Europäisches Patentamt / European Patent Office / Office européen des brevets

(11) EP 4 502 590 A1

(12) EUROPEAN PATENT APPLICATION
published in accordance with Art. 153(4) EPC

(43) Date of publication:
05.02.2025 Bulletin 2025/06

(21) Application number: 23772752.4

(22) Date of filing: 25.01.2023

(51) International Patent Classification (IPC):
$G01N\ 27/416^{(2006.01)}$    $G01N\ 27/26^{(2006.01)}$
$G01N\ 27/28^{(2006.01)}$

(52) Cooperative Patent Classification (CPC):
G01N 27/26; G01N 27/28; G01N 27/416

(86) International application number:
PCT/JP2023/002294

(87) International publication number:
WO 2023/181620 (28.09.2023 Gazette 2023/39)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.03.2022 JP 2022049361

(71) Applicant: HITACHI HIGH-TECH CORPORATION
Tokyo 105-6409 (JP)

(72) Inventors:
• KISHIOKA Atsushi
  Tokyo 100-8280 (JP)
• MATSUSHITA Yufuku
  Tokyo 100-8280 (JP)
• MIYAKE Masafumi
  Tokyo 105-6409 (JP)

(74) Representative: Strehl Schübel-Hopf & Partner
Maximilianstrasse 54
80538 München (DE)

(54) **ELECTROLYTE ANALYSIS DEVICE**

(57) Provided is an electrolyte analysis device capable of determining a type or concentration of an interfering ion more easily.

The electrolyte analysis device having an ion-selective electrode and using potential measurement includes a storage unit configured to store a relationship of a potential change over time with respect to an interfering ion, and an interfering ion analysis unit configured to detect an influence of the interfering ion based on the potential change over time obtained from the ion-selective electrode while a specimen solution is stationary after the specimen solution comes in contact with the ion-selective electrode.

FIG. 5

EP 4 502 590 A1

**Description**

Technical Field

The present invention relates to an electrolyte analysis device.

Background Art

An electrolyte concentration analysis device that conducts an analysis with potential measurement (potentiometric) obtains a potential generated at a solid-liquid interface between an ion sensitive membrane of an ion-selective electrode (ion electrode) and a sample solution (specimen) and converts the potential into a concentration to conduct an analysis. The potential generated at the solid-liquid interface between the ion sensitive membrane and the sample solution changes depending on an activity amount of the ions to be measured in the sample solution (Nernst response). Due to the simplicity of the measurement, the ion-selective electrode is used for electrolyte concentration analysis in liquid samples, such as food, water and wastewater in factories, and biological samples. When the potential is obtained, the sample solution is brought into contact with the ion sensitive membrane of the electrode, and the potential is measured at a stable timing.

Usually, since the ion-selective electrode measures one specific type of ion, the same number of electrodes are required to detect plural types of ions. Further, the ion-selective electrode is preferably an electrode having enhanced ion selectivity so as not to be easily affected by ions other than the ion to be measured. However, an anion-selective electrode is technically difficult to improve ion selectivity, and is more susceptible to interfering ions than a cation-selective electrode.

An electrolyte concentration measurement device mounted on a biochemical automatic analysis device will be described as an example. Since a specimen to be analyzed is a biological sample such as serum, ion species and ion concentrations in the specimen are determined to some extent. It is required to analyze a relatively small difference in ion concentration with high throughput. The three types of electrolyte items that are often conducted in the biochemical analysis are Na, K, and Cl ions.

With respect to cations such as Na ions and K ions, an ion sensitive membrane having high ion selectivity is known, and is hardly affected by interfering ions. On the other hand, it is technically difficult to make an ion sensitive membrane that is hardly affected by both hydrophilic ions and lipophilic ions in an anion-selective electrode such as Cl ions. Therefore, the analysis accuracy is improved by devising a measurement method.

An example of such a method is a method for performing calibration using a matrix under a standard condition of a specimen containing interfering ions. However, even with this method, in the case of a specimen having a concentration or type of interfering ions different from usual, the influence of the interfering ions cannot be canceled. Accordingly, the ion concentration analysis result may be affected. Examples of such a specimen include lyophilized control serum (having a low concentration of bicarbonate ion ($HCO_3^-$)) and a patient specimen to which medication containing ions that are not usually present is being administered.

PTL 1 to PTL 3 disclose techniques that can be used for such a specimen in which the type and concentration of interfering ions are irregular.

PTL 1 discloses a technique in which "in addition to a base electrode which is a chlorine ion-selective electrode, a first auxiliary electrode having a greater selection coefficient for lipophilic ions than that of the base electrode and a second auxiliary electrode having a greater selection coefficient for hydrophilic ions are provided, and when a measurement value of the auxiliary electrode is greater than a measurement value of the base electrode and a difference exceeds a set value, an alarm is generated".

PTL 2 discloses a technique such that "a solution is brought into contact with a plurality of electrodes, each electrode is configured to generate a signal in response to sensing of a selected ion in the solution. Ion interference between the selected ion and another ions detected in a solution in one of electrodes and/or electrode interference between the electrodes are calculated using a neural network algorithm. The ion interference and/or the electrode interference is compensated based on a result of comparing training data indicating known ion concentrations".

PTL 3 discloses a technique such that "the concentration of a target ion contained in a sample is calculated by using the result of calculating a selection coefficient of an ion selection electrode and the result of measuring the concentration of coexisting ions contained in the sample".

Citation List

Patent Literature

PTL 1: JP 2000-121595 A
PTL 2: US 2013/0304395 A
PTL 3: WO2019/163281 A

Summary of Invention

Technical Problem

The conventional technique has a problem that the type or concentration of interfering ions are difficult to determine. For example, in the techniques of PTL 1 and PTL 2, measurement values at a plurality of electrodes having different selectivity are required. In addition, for example, in PTL 3, the concentration of interfering ions needs to be obtained with another analysis method.

The present invention has been made to solve such a problem. An object of the present invention is to provide an electrolyte analysis device capable of more easily determining the type or concentration of interfering ions.

Another object is to provide an electrolyte analysis device capable of making such a determination with one electrode.

Solution to Problem

An electrolyte analysis device of the present invention as one example having an ion-selective electrode and using potential measurement includes

a storage unit configured to store a relationship of a potential change over time with respect to an interfering ion, and an interfering ion analysis unit configured to detect an influence of the interfering ion based on the potential change over time obtained from the ion-selective electrode while a specimen solution is stationary after the specimen solution comes in contact with the ion-selective electrode.

This specification contains the disclosure of Japanese Patent Application No. 2022-049361, which is the basis of the priority of the present application.

Advantageous Effects of Invention

The electrolyte analysis device of the present invention can more easily determine the type or concentration of interfering ions. Further, according to one example, such a determination can be made with one electrode.

Brief Description of Drawings

FIG. 1 is a block diagram illustrating an overall configuration of a flow-type electrolyte concentration measurement device according to a first embodiment of the present invention.
FIG. 2 is a flowchart at a time of activating the device according to the first embodiment of the present invention.
FIG. 3 is a flowchart at a time of analyzing the concentration of an electrolyte according to the first embodiment of the present invention.
FIG. 4 is a diagram illustrating a representative potential waveform according to the first embodiment of the present invention.
FIG. 5 is a diagram illustrating potential waveforms when measuring various solutions according to the first embodiment of the present invention.
FIG. 6 is a schematic diagram illustrating a phenomenon mechanism used in the present invention.
FIG. 7 is a block diagram of an experimental device for verifying the principle of the present invention.
FIG. 8 is a diagram illustrating an experimental result in the experimental device for verifying the principle of the present invention.
FIG. 9 is a schematic diagram illustrating a phenomenon mechanism used in the present invention.
FIG. 10 is a flowchart of analyzing the concentration of an electrolyte according to a second embodiment of the present invention.
FIG. 11 is a flowchart of analyzing the concentration of an electrolyte according to a third embodiment of the present invention.

Description of Embodiments

The present inventors have conducted research and development on a method for detecting and reducing the influence of interfering ions in order to achieve more reliable analysis in an electrolyte concentration measurement device. As a result, the present inventors have found that interfering ions can be detected more easily (for example, without installing an additional electrode or sensor), although the detection has been conventionally difficult. Further, a device that conducts more stable analysis utilizing information about the interfering ions has been implemented.

Hereinafter, embodiments of the present invention will be described with reference to the drawings.

[First embodiment]

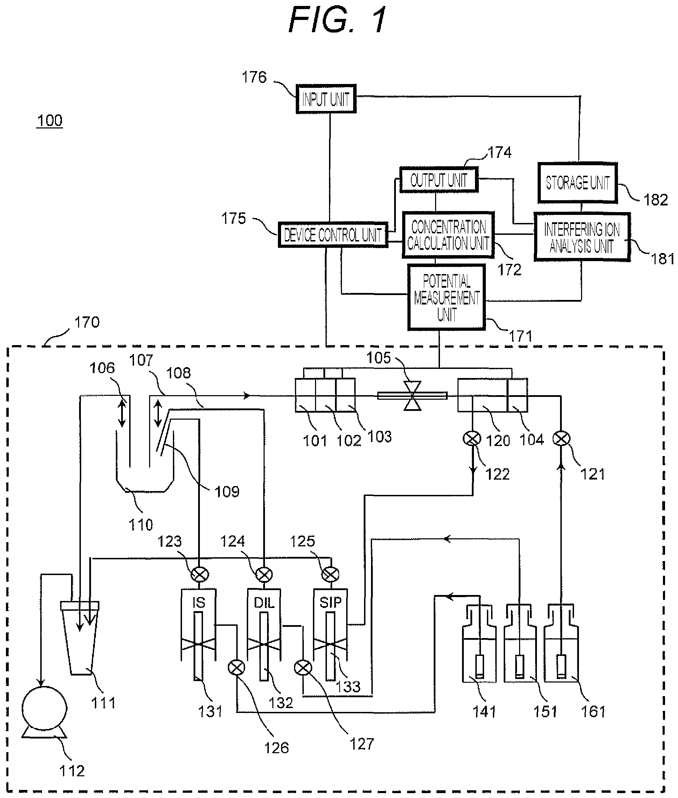

FIG. 1 is a schematic view illustrating one example of a flow-type electrolyte concentration measurement device according to a first embodiment of the present invention. The electrolyte concentration measurement device 100 is an electrolyte analysis device having an ion-selective electrode and using potential measurement. The characteristic of the present embodiment is, in particular, a measurement result of a Cl ion electrode in which ions to be measured by the electrolyte concentration measurement device 100 are anions, and the interfering ions of anions can be detected.

The device of the present embodiment is a device that analyzes the concentrations of three types of ions including Na, K, and Cl ions. The device outputs the analysis results of respective ion concentrations and a determination result as to whether the Cl ion concentration measurement is affected by interfering ions.

Note that counter ions with respect to fixed charges in a sensitive membrane is referred to as main ions (Cl ions in the case of a Cl ion electrode). Ion species having charges of the same sign as that of the main ions and other than the main ions are referred to as interfering ions.

The electrolyte concentration measurement device 100 includes a measurement unit 170, a potential measurement unit 171, a concentration calculation unit 172, an output unit 174, a device control unit 175, an input unit 176, an interfering ion analysis unit 181, and a storage unit 182.

The measurement unit 170 includes, as ion-selective electrodes, three types of electrodes including a Cl ion electrode 101 (chlorine ion electrode), a K ion electrode 102 (potassium ion electrode), and a Na ion electrode 103 (sodium ion electrode). Further, the measurement unit 170 includes a reference electrode 104. As a sensitive membrane of the Cl ion electrode 101, an ion sensitive membrane based on an anion exchange membrane having a high-density fixed charge is used.

In the present embodiment, the ion-selective electrodes are flow-type ion-selective electrodes. The use of the flow-type ion-selective electrodes are suitable because a potential change immediately after a solution is stationary is easily measured.

A dilution tank 110 temporarily stores a diluted specimen in which a specimen dispensed from a specimen nozzle (not illustrated) and a diluent dispensed from a diluent supply nozzle 108 are mixed, or an internal standard solution dispensed from an internal standard solution supply nozzle 109. A sipper nozzle 107 descends into the dilution tank 110, and the diluted specimen or internal standard solution in the dilution tank 110 is introduced into channels of ion-selective electrodes (the Cl ion electrode 101, the K ion electrode 102, and the Na ion electrode 103: hereinafter, the same applies) by a sipper syringe 133. Further, a reference electrode solution is introduced from a reference electrode solution bottle 161 into a channel of the reference electrode 104 using the sipper syringe. During this time, a vacuum aspiration nozzle 106 descends, and the diluted specimen or the internal standard solution remaining in the dilution tank 110 is aspirated and discharged to a waste tank 111. A vacuum pump 112 is connected to the waste tank 111.

Here, the detailed operation of the mechanism portion for introducing a solution into the channel of the electrode will be described. First, when the solution in the dilution tank is introduced into the channels of the ion-selective electrodes, a solenoid valve 121 and a solenoid valve 125 are closed, and a pinch valve 105 and a solenoid valve 122 are opened. Then, the sipper nozzle 107 is descended into the dilution tank 110, and the sipper syringe 133 is pulled. Subsequently, when a reference electrode solution is introduced into the channel of the reference electrode 104, the solenoid valve 121 is opened, the pinch valve 105 is closed, and the sipper syringe 133 is pulled. As a result, the reference electrode solution is introduced from the reference electrode solution bottle 161 into the channel of the reference electrode 104. Further, in order to discharge the solution accumulated in the sipper syringe, the solenoid valve 122 is closed, the solenoid valve 125 is opened, and the sipper syringe 133 is pushed.

In addition, a solenoid valve 123, a solenoid valve 124, a solenoid valve 126, a solenoid valve 127, and an internal standard solution syringe pump 131 are provided.

Note that the reference electrode solution introduced into the channel of the reference electrode 104 and the solution introduced into the ion-selective electrodes come in contact with each other at a liquid junction 120. The ion-selective electrodes and the reference electrode 104 are electrically connected to each other through the solution. In this state, an electromotive force (potential) between the reference electrode 104 and each ion-selective electrode changes depending on the concentration of ions to be measured in the solution introduced into the channel of each ion-selective electrode.

The potential information during the series of the analysis operations is acquired by the potential measurement unit 171. The interfering ion analysis unit 181 receives, from the potential measurement unit 171, potential waveforms in the solution stationary state after the solution is introduced into the channels of the ion-selective electrodes, and analyzes the influence of interfering ions using the information stored in the storage unit 182.

The concentration calculation unit 172 receives the measured potential at a stable timing suitable for calculation of the concentration from the potential measurement unit 171, and calculates the concentration of ions to be measured.

The output unit 174 displays the operation status of the device received from the device control unit 175 and the analysis results in the concentration calculation unit 172 and the interfering ion analysis unit 181. An operator can input specimen information, various parameters, a device operation command, and the like, through the input unit 176. Details of a calculation method will be described later.

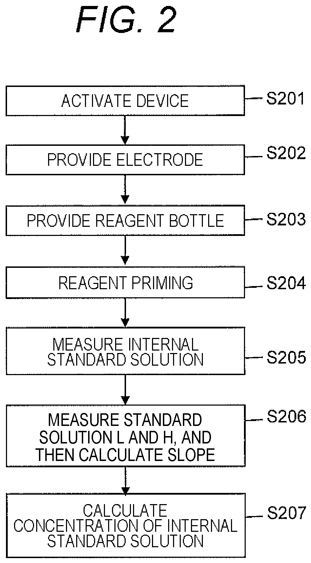

Next, a flow at the time of activating the electrolyte concentration measurement device 100 will be described with reference to FIG. 2. First, a procedure at the activation time will be described. The electrolyte concentration measurement device 100 is activated (S201), an electrode is provided (S202), and a reagent bottle is provided (S203). Reagent priming is performed so that a new reagent is supplied to the insides of the syringe pump and the channels to fill the insides (S204). Continuous measurement of the internal standard solution is performed to check that the potentials of the electrodes are stable (S205). In order to obtain a calibration curve of each ion-selective electrode, two types of standard solutions having known concentrations are measured, and slopes are calculated (S206). Subsequently, the concentration of the internal standard solution is calculated (S207).

Here, specific operations in S206 and S207 will be described. After the known low-concentration standard solution is dispensed into the dilution tank 110 by the dispensing nozzle (not illustrated), the diluent in the diluent bottle 151 is dispensed into the dilution tank using a diluent syringe pump 132. In this manner, the known low-concentration standard solution is diluted at a set ratio D. The diluted known low-concentration standard solution in the dilution tank 110 is aspirated from the sipper nozzle 107, and is introduced into the channel of each ion-selective electrode.

Thereafter, the reference electrode solution is introduced into the channel of the reference electrode 104 from the inside of the reference electrode solution bottle 161. At the liquid junction, the reference electrode solution and the diluted known low-concentration standard solution come in contact with each other. The potential measurement unit 171 measures each electromotive force between each ion-selective electrode and the reference electrode 104 while the solution is stationary immediately after the diluted standard solution is introduced into each electrode channel.

Meanwhile, the solution remaining in the dilution tank 110 is aspirated by the vacuum aspiration nozzle 106. Then, the internal standard solution in the internal standard solution bottle 141 is dispensed into the dilution tank 110. The internal standard solution in the dilution tank 110 is aspirated from the sipper nozzle 107 to fill the channel of each ion-selective electrode. The reference electrode solution is introduced from the inside of the reference electrode solution bottle 161 into the channel of the reference electrode 104.

The potential measurement unit 171 measures the electromotive force of each electrode while the solution is stationary immediately after the internal standard solution is introduced into each electrode channel. Further in the meantime, the solution remaining in the dilution tank 110 is aspirated by the vacuum aspiration nozzle. Then, the known high-concentration standard solution is dispensed into the dilution tank 110 by the dispensing nozzle (not illustrated) . Thereafter, the diluent in the diluent bottle 151 is dispensed into the dilution tank using the diluent syringe pump 132, and the known high-concentration standard solution is diluted at the set ratio D.

The diluted known high-concentration standard solution in the dilution tank is aspirated from the sipper nozzle, and is introduced into the channel of each ion-selective electrode. Thereafter, the reference electrode solution is introduced into the channel of the reference electrode 104 from the inside of the reference electrode solution bottle 161. At the liquid junction, the reference electrode solution and the diluted known high-concentration standard solution come in contact with each other.

The potential measurement unit 171 measures each electromotive force between each ion-selective electrode and the reference electrode 104 while the solution is stationary immediately after the diluted standard solution is introduced into each electrode channel. Meanwhile, the solution remaining in the dilution tank 110 is aspirated by the vacuum aspiration nozzle. Then, the internal standard solution in the internal standard solution bottle 141 is dispensed into the dilution tank 110. The internal standard solution in the dilution tank is aspirated from the sipper nozzle to fill the channel of each ion-selective electrode. Then, the reference electrode solution is introduced from the inside of the reference electrode solution bottle 161 into the channel of the reference electrode 104.

The potential measurement unit 171 measures the electromotive force of each electrode while the solution is stationary immediately after the internal standard solution is introduced into each electrode channel. Further, the solution remaining in the dilution tank 110 is aspirated by the vacuum aspiration nozzle.

As described above, the potential measurement unit 171 can obtain potential waveforms while the solutions are stationary immediately after introduction of the three types of solutions including the low-concentration standard solution, the high-concentration standard solution, and the internal standard solution. The concentration calculation unit 172 receives potential values (potential differences) in a time domain where the potentials are the most stable among the potential waveforms obtained by the potential measurement unit 171, and sets the potential value as the measured electromotive forces (EMFs) of the solutions. Note that according to the above sequence, the waveform of the internal standard solution can be obtained twice, and in principle, the same value is obtained from the solution having the same composition. In a case where the same value cannot be obtained, it is considered that this is because of an influence of a remaining solution measured previously. Using such information, a correction may be made at the time of calculating a slope sensitivity or the concentration of the internal standard solution. In addition, such information can be used as an alarm of a device state or a time index of device maintenance.

The concentration calculation unit 172 calculates a slope sensitivity SL corresponding to a calibration curve based on the electromotive force received from the potential measurement unit 171 using the following Equation (1).

(A) Slope sensitivity

$$SL = (EMF_H - EMF_L)/(LogC_H - LogC_L) \ ... \ Equation \ (1)$$

wherein

SL: slope sensitivity
$EMF_H$: measured electromotive force of known high-concentration standard solution
$EMF_L$: measured electromotive force of known low-concentration standard solution
$C_H$: Known concentration value of high-concentration standard solution
$C_L$: Known concentration value of low-concentration standard solution

The above operation is referred to as calibration. Note that the slope sensitivity SL corresponds to a portion of "$2.303 \times (RT/zF)$" in the following Nernst equation.

$$Nernst \ equation: \ E = E0 + 2.303 \times (RT/zF) \times log(f \times C)$$

(wherein E0: constant potential determined by a measurement system, z: valence of ions to be measured, F: Faraday constant, R: gas constant, T: absolute temperature, f: activity coefficient, and C: ion concentration)

The slope sensitivity SL can be obtained by calculation from a temperature and the valence of ions to be measured. In the present embodiment, the slope sensitivity SL specific to the electrode is obtained by the above-described calibration in order to further improve the analysis accuracy.

Subsequently, the concentration of the internal standard solution is calculated based on the slope sensitivity and the electromotive force of the internal standard solution.

(B) Concentration of internal standard solution

$$C_{IS} = C_L \times 10^a \ \ \ \ \ \ ... \ Equation \ (2)$$

$$a = (EMF_{IS} - EMF_L)/SL \ \ ... \ Equation \ (3)$$

wherein

$C_{IS}$: Concentration of internal standard solution
$EMF_{IS}$: Electromotive force of internal standard solution

An example of a specific calibration method has been described above. Instead of this procedure, a different procedure may be used as long as two or more types of solutions having different ion concentrations can be introduced into the channel and the electromotive force can be measured. Note that the standard solution may contain interfering ions such as bicarbonate ions. Further, a standard sample having a composition similar to that of a serum sample or a urine sample may be measured, and the calibration correction may be further performed.

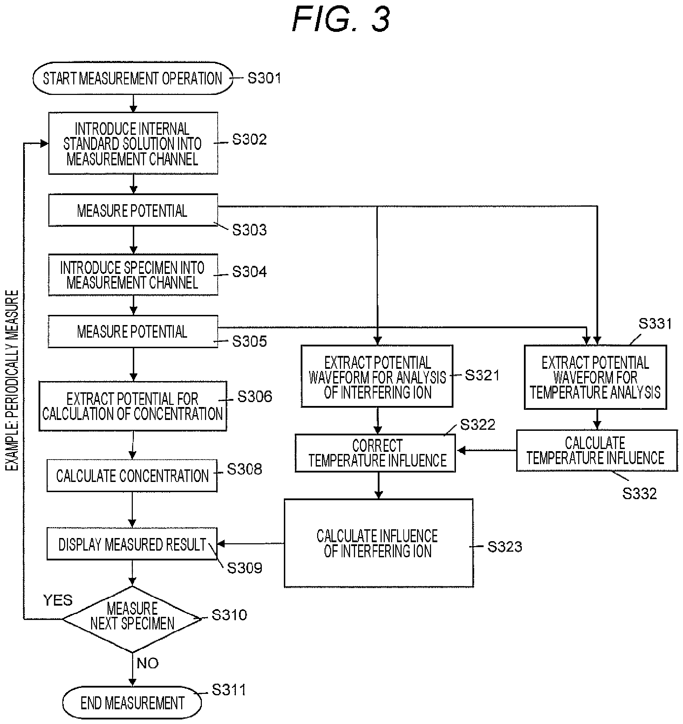

After the calibration, serum, urine, or the like is analyzed as a specimen. Next, a flow of continuous analysis in the present embodiment will be described with reference to FIG. 3.

Specifically, when the measurement operation is started (S301), the internal standard solution in the internal standard solution bottle 141 is dispensed into the dilution tank. The internal standard solution in the dilution tank is aspirated from the sipper nozzle 107 to fill the channel of each ion-selective electrode. Then the reference electrode solution is introduced from the inside of the reference electrode solution bottle 161 into the channel of the reference electrode 104 (S302).

The potential measurement unit 171 measures the electromotive force of each electrode while the solution is stationary immediately after the internal standard solution is introduced into the electrode channel (S303). Further in the meantime, after the solution remaining in the dilution tank 110 is aspirated by the vacuum aspiration nozzle, the specimen is dispensed into the dilution tank 110 by the dispensing nozzle (not illustrated). Thereafter, the diluent in the diluent bottle 151 is dispensed into the dilution tank using the diluent syringe pump 132, and the specimen is diluted at the set ratio D.

The diluted specimen (sample) in the dilution tank 110 is aspirated from the sipper nozzle to fill the channel of each ion-selective electrode. The reference electrode solution is introduced from the inside of the reference electrode solution bottle 161 into the channel of the reference electrode 104 (S304).

The potential measurement unit 171 measures the electromotive force of each electrode while the solution is stationary immediately after the sample is introduced into the electrode channel (S305). Further, the solution remaining in the dilution tank is aspirated by the vacuum aspiration nozzle.

Next, the concentration calculation unit 172 extracts a potential value for concentration calculation from the potential measurement unit 171 (S306). The concentration calculation unit 172 calculates the concentration of the sample based on the slope sensitivity and the concentration of the internal standard solution using the following Equations (4) and (5) (S308).

(C) Concentration of sample

$$C_S = C_{IS} \times 10^b \qquad \ldots \text{Equation (4)}$$

$$b = (EMF_{IS} - EMF_S)/SL \quad \ldots \text{Equation (5)}$$

wherein

$C_S$: Sample concentration

$EMF_S$: Measured electromotive force of sample

The device according to the present embodiment the calculation and correction of the specimen measurement based on the value of the measurement potential of the internal standard solution having a constant concentration measured before the specimen measurement. Thereby, the device according to the present embodiment can achieve accurate measurement even if a gentle potential fluctuation (potential drift phenomenon), such as a change in a membrane surface or a temperature change, occurs. Note that the measured potentials of the internal standard solution before and after the specimen measurement may be used as well as the measured potential before the specimen measurement.

Apart from the potential value for concentration calculation, the interfering ion analysis unit 181 extracts a potential waveform for interfering ion analysis from the potential measurement unit 171 (S321).

Further, the interfering ion analysis unit 181 extracts a potential waveform for temperature analysis from the potential measurement unit 171 (S331). The interfering ion analysis unit 181 corrects a temperature influence in the potential waveform for interfering ion analysis extracted in S321 (S322) using the result of calculating the temperature influence (S332), and calculates the influence of interfering ions (S323). In this manner, after the specimen comes in contact with the ion-selective electrode, the interfering ion analysis unit 181 detects the influence of the interfering ions based on a potential change over time obtained from the ion-selective electrode while the specimen solution is stationary.

The interfering ion analysis unit 181 then displays the interfering ion influence detected result on the output unit 174 together with a concentration result of the ions to be measured (S309). In particular, the output unit 174 displays the detection result of the influence of the interfering ions. As a result, a user can know the influence of the interfering ions.

In a case where a next specimen is measured, the processing returns to S302 again (S310), otherwise the measurement is ended (S311).

Note that in the device according to the present embodiment, in S321 and S331, the potential waveform is extracted not only at the time of the specimen measurement but also at the time of measuring the internal standard solution, and the waveform is used to check the variation of the device state and the like.

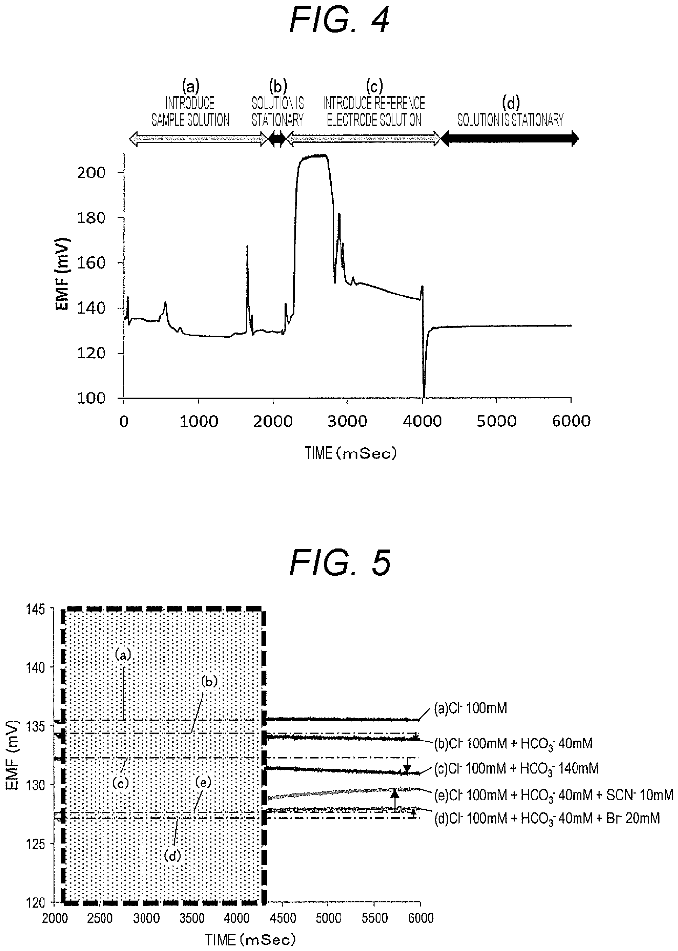

Here, a method for calculating the influence of interfering ions will be described. FIG. 4 illustrates a potential waveform in the entire time domain in one cycle at the time of measuring the sample solution in the present embodiment. The time domain is mainly divided into (a) the time of introducing a sample solution, (b) the time a solution is stationary, (c) the time of introducing the reference electrode solution, and (d) the time a solution is stationary.

(a) At the time of introducing a sample solution or (c) at the time of introducing a reference electrode solution, vibration, liquid flow, and electrical noise is caused due to the operation of the solenoid valves, the pinch valve, the syringe pump, or the like, and thus the potential waveform jitters. As the potential waveform (S321) for the interfering ion analysis of the present embodiment, the time domains of (b) and (d) at the time a solution is stationary are extracted. Note that the potential waveform may not be a continuous waveform or a waveform including values at a large number of times. The potential waveform may include potential values at two or more points of different times at the time the solution is stationary.

The potential waveform obtained in S321 is subjected to temperature influence correction described later. The storage unit 182 stores a correlation between the potential waveform and the interfering ions in this time domain. The influence of the interfering ions can be calculated by analyzing the temperature-corrected potential waveform using the information in the storage unit.

The storage unit 182 stores a relationship between a potential change over time and the interfering ions. For example, the interfering ion analysis unit 181 specifies the type of interfering ions based on the direction of change in the potential waveform. As a specific example, in a case where a temporal gradient of the potential waveform is positive, a determination is made that lipophilic interfering ions such as $Br^-$ and $SCN^-$ are contained. In a case where the temporal gradient is negative, a determination is made that hydrophilic interfering ions such as $HCO_3^-$ are contained. In this way, the type of interfering ions can be identified.

In addition, by collating with the information stored in the storage unit 182, the type or concentration of interfering ions can be estimated based the magnitude of the gradient. In a case where the type of interfering ions is known, the interfering ion analysis unit 181 calculates the concentration of the interfering ions in accordance with the magnitude of the gradient based on the gradient of the change in the potential waveform. In this way, the concentration of the interfering ions can be calculated.

Note that the information stored in the storage unit 182 may be information input before shipment of the device. The information can be input in accordance with the state of the device used by the user and the characteristics of the electrodes through the input unit 176.

For example, the input unit 176 may be used for inputting information about a specimen to the storage unit 182. The information about the specimen may indicate, for example, the type of a medication that may be contained in the specimen. The storage unit 182 may store the type of a medication and the type of interfering ions contained in the medication in association with each other. In this way, since the type of the interfering ions is specified by inputting the type of the medication, the concentration of the interfering ions can be estimated more accurately.

Here, hydrophilicity and lipophilicity of ions will be described. The minimum concentration required for salting out protein varies with ionic species, and its order is known as the Hofmeister series. With regard to the anions, the order is as follows.

$$SO_4^- > HCO_3^- > Cl^- > Br^- > NO_3^- > I^- > SCN^-$$

The left side is called a hydrophilic ion and the right side is called a lipophilic ion. In general, the ion selectivity of the Cl ion electrode tends to increase in the reverse order of the Hofmeister series. That is, it tends to easily respond to $Br^-$ or the like on the more lipophilic side than $Cl^-$, and hardly respond to $HCO_3^-$ or the like on the more hydrophilic side than $Cl^-$.

Here, FIG. 5 illustrates a measurement example in the present embodiment. Illustrated potential waveforms are obtained when several aqueous solutions having compositions different from each other are prepared as simulated specimen and are measured. The compositions of the aqueous solutions are as follows.

(a) $Cl^-$ 100 mM
(b) $Cl^-$ 100 mM + $HCO_3^-$ 40 mM
(c) $Cl^-$ 100 mM + $HCO_3^-$ 140 mM
(d) $Cl^-$ 100 mM + $HCO_3^-$ 40 mM + $Br^-$ 20 mM
(e) $Cl^-$ 100 mM + $HCO_3^-$ 40 mM + $SCN^-$ 10 mM

The vertical axis represents the potential. The horizontal axis represents the time. A time domain surrounded by a thick dotted line is a domain where the potential is not stabilized by the operation of a drive mechanism. Other domains are time domains where each solution is introduced into the electrode channel and each solution is stationary.

As described above, all the specimen measured here have the same Cl ion concentration. It is found that the influence of interfering ions varies depending on the selectivity of each ion of the electrode. It is also found that the influence is relatively small for $HCO_3^-$ as hydrophilic ions, but the influence is great even at a low concentration for lipophilic ions such as Br⁻.

As in the conventional device, for example, when the potential for concentration calculation is obtained at 6000 ms when the potential is relatively stable, different values are obtained. When the concentration is calculated based on this potential, a concentration different from the true value is output as the Cl ion concentration if interfering ions are contained, but a determination cannot be made whether interfering ions are contained. Therefore, in the conventional device, it has been avoided to analyze a specimen in which the state of interfering ions in blood is changed during medication or the like. On the contrary, in the present embodiment, it is also possible to output whether the calculated value of the Cl ion concentration is affected by the interfering ions at the same time to notify the user.

This method will be described. A dot-and-dash line horizontally extended from the potential value at 2000 ms of each specimen is shown in FIG. 5. The deviation from the dot-and-dash line represents a potential change over time.

When the potential at 6000 ms is seen, for the specimen (a), the potential has almost no deviation with respect to the dot-and-dash line. For the specimen (b), the potential has a slightly low value with respect to the dot-and-dash line. For the specimen (c), the potential has a significantly low value with respect to the dot-and-dash line. On the other hand, the values of the specimens (d) and (e) are high with respect to the dot-and-dash line.

The reason for this is as follows. For the specimen (a), since only Cl⁻ is contained and interfering ions are not contained, the potential does not change with the lapse of time while the solution is stationary. Whereas for the specimen (b), 40 mM of $HCO_3^-$ as hydrophilic ions is contained, and thus a slightly negative slope is exhibited. For the specimen (c), 140 mM of $HCO_3^-$ is contained, and thus a greater negative slope is exhibited. On the other hand, the specimens (d) and (e) exhibit a positive slope because not only $HCO_3^-$ 40 mM but also lipophilic ions Br⁻ and SCN⁻ are contained.

The storage unit 182 stores such information about the correlation among the ion species, the concentration, and the potential. As a result of analyzing the obtained potential waveform in the interfering ion analysis unit 181, when the deviation amount exceeds a certain value, the output unit outputs the influence of the interfering ions.

For example, since serum usually contains 30 to 40 mM $HCO_3^-$, the slope of the curve of the specimen (b) can be used as a reference. When consideration is given as a measure of the specimen (b), the specimen having a slope as in the specimen (b) is normal. The output unit outputs the possibility that $HCO_3^-$ is significantly less than usual for the specimen having a flat waveform of the specimen (a). The output unit outputs the possibility that hydrophilic ions are contained more than usual for the specimen having a waveform with a negative slope as in the specimen (c). The output unit outputs the possibility that lipophilic ions are contained for the specimen having a waveform with a positive slope as in the specimens (b) and (e). When seeing such a result, the user can review the identity of the specimen and determine the reliability of a Cl ion concentration analysis value output at the same time.

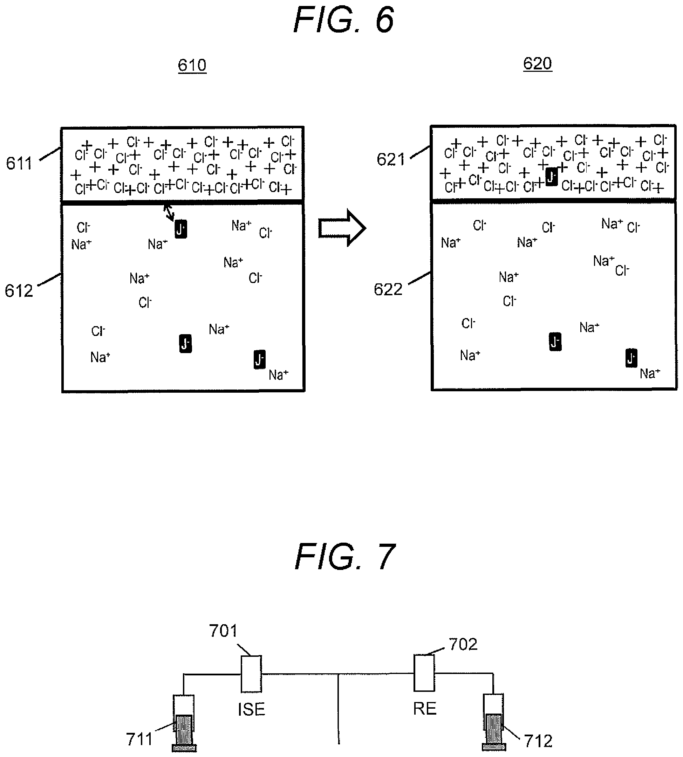

The reason why the potential changes over time when the specimen containing interfering ions is measured will be described with reference to the schematic diagram of FIG. 6.

Reference numerals 611 and 621 denote ion sensitive membranes. Reference numerals 612 and 622 denote sample solutions. Reference numeral 610 denotes a state immediately after the sample solution is introduced. Reference numeral 620 denotes a state after the solution is stationary.

A Cl ion sensitive membrane of the present embodiment is based on an ion exchange membrane. Immobilized cations with a high concentration exist in the sensitive membrane 611, and Cl⁻ with a high concentration exists as a counter anion. Therefore, in a case of contact with the sample solution containing interfering ions J⁻, ion exchange between the interfering ions J⁻ in the sample solution and Cl⁻ in the membrane occurs quickly. This is a phenomenon that occurs characteristically when a membrane having high-density fixed charges is used as the ion sensitive membrane.

For example, in a case where a sample solution containing $HCO_3^-$ is introduced, that is, J⁻ is $HCO_3^-$, exchange of $HCO_3^-$ in the sample solution and Cl⁻ in the membrane occurs immediately after the introduction. In a case where the solution is stationary, J⁻ ions in the sample solution near the membrane are replaced by Cl⁻ ions.

The Cl ion electrode has higher selectivity with respect to Cl⁻ than $HCO_3^-$ and easily responds to Cl⁻. Therefore, when $HCO_3^-$ in the sample solution present near the membrane surface is exchanged for Cl⁻, the Cl ion electrode senses that the ion concentration of the sample solution is high. Since the slope sensitivity is negative, the potential changes to decrease as the exchange reaction of J⁻ in the sample solution with Cl⁻ proceeds. When the same sample solution is introduced again, the sample solution is refreshed and the similar phenomenon occurs again.

Although $HCO_3^-$ also flows into the membrane side the influence on the membrane side is small because Cl⁻ with high density exists in the membrane.

In a case where a sample solution containing Br⁻ or SCN⁻ is introduced, that is, J⁻ is Br⁻ or SCN⁻, ion exchange is similarly performed between the sample solution and the membrane. Since the Cl ion electrode has higher selectivity for Br⁻ or SCN⁻ than Cl⁻, when Br⁻ or SCN⁻ in the sample solution present near the membrane surface is exchanged for Cl⁻, the Cl ion electrode senses that the ion concentration in the sample solution is low. Since the slope sensitivity is negative, the potential changes to increase.

In order to more easily understand this phenomenon, a result of verification of the principle with a simple experimental system will be described. FIG. 7 illustrates a block diagram of the experimental device. A Cl ion electrode 701 is present in a left channel. A reference electrode 702 is provided in a right channel via a solution drop portion. From the right channel, the reference electrode solution can be introduced into the reference electrode channel by a syringe 712 filled with the reference electrode solution. From the left channel, the sample solution can be introduced into the Cl ion electrode channel by a sample solution syringe 711.

The solution was introduced and allowed to stand still for about 3 minutes. Then, a next sample solution was introduced and then allowed to stand still for another 3 minutes. During the series of measurement, the potential between the Cl ion electrode channel and the reference electrode was continuously measured.

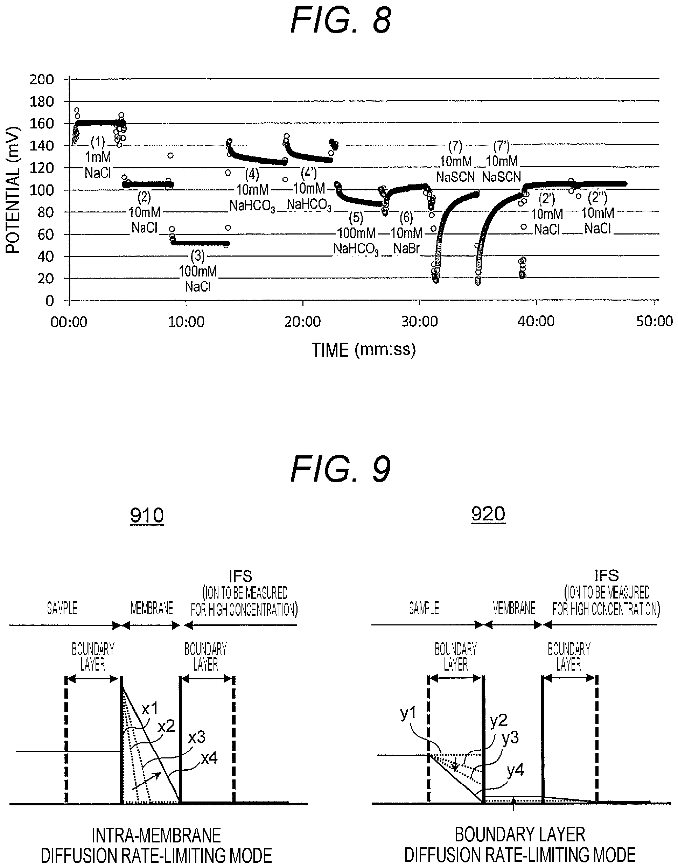

FIG. 8 illustrates the potential measured results in this experiment. A vertical axis represents the potential, and a horizontal axis represents time. For each of the NaCl aqueous solutions having different concentrations of (1) to (3), a stable potential is exhibited over time. For the (4) 10 mM $NaHCO_3$ aqueous solution, the potential sharply decreased immediately after the solution is stationary, and the change gradually became gentle. (4') When a $NaHCO_3$ aqueous solution having the same concentration was introduced again, a similar curve was drawn starting from substantially the same potential as in (4). (5) When a 100 mM $NaHCO_3$ aqueous solution was introduced, the potential similarly changed in a negative direction. On the other hand, (6) in a case where a 10 mM NaBr aqueous solution was introduced, the potential rapidly increased immediately after the solution was stationary contrary to the $NaHCO_3$ aqueous solution, and this change tended to gradually be gentle. (7) A 10 mM NaSCN aqueous solution exhibited a more remarkable potential change. (7') The phenomenon was reproduced when the 10 mM NaSCN aqueous solution was introduced again. Finally, when 10 mM NaCl aqueous solutions of (2') and (2") were introduced, the potential was almost the same as that of (2). As described above, the results supporting the phenomenon described with reference to FIG. 6 were obtained.

In general, in the ion-selective electrode, since the lower limit of the measured concentration is deteriorated, it is undesirable to generate ion flux from the ion sensitive membrane. However, in the present embodiment, this phenomenon is reversely used, and not only the concentration of the ions to be measured but also the concentration of interfering ions can be detected with one electrode.

A situation where the above phenomenon occurs will be described with reference to a schematic diagram of FIG. 9. It is considered that the following two different phenomena mainly occur depending on the magnitude relationship between a diffusion rate in the membrane and the solution and an ion exchange reaction rate. These phenomena are shown in 910 and 920 of FIG. 9.

Reference numerals 910 and 920 schematically indicate changes in the concentration of interfering ions in each domain in a case of an intra-membrane diffusion rate-limiting mode and a case of a boundary layer diffusion rate-limiting mode, respectively. The ion-selective electrode includes an inner solution (Inner Filling Solution; IFS). The internal solution contains ions with a high concentration to be measured. Here, the high concentration means an ion concentration or more contained in the sample solution to be measured.

In 910 and 920 of FIG. 9, a region sandwiched between two thick vertical solid lines represents an ion sensitive membrane ("membrane"). The left side of the membrane represents a sample solution ("sample"). The right side of the membrane represents an internal solution ("IFS").

A region near the membrane of each of the sample solution and the internal solution, that is, a region closer to each membrane than a vertical broken line is a boundary layer of each of the solutions, that is, a region where solution flow does not occur and only diffusion occurs predominantly.

In a case where the ion diffusion in the membrane is sufficiently slower than the ion diffusion of the boundary layer in the solution, the distribution of the interfering ion concentration is the intra-membrane diffusion rate-limiting mode 910. That is, in a case where the sample solution containing interfering ions comes in contact with the membrane, the ions in the solution are exchange for ions in the membrane. Since diffusion in the membrane is slow, ions on the surface of the membrane is exchanged for the interfering ions. The ratio and the like vary depending on selectivity and the like. Since the diffusion in the sample solution is sufficiently faster than in the membrane, the change in the ion concentration in the boundary layer is small. In an electrode including a so-called solution film-type ion sensitive membrane in which a quaternary ammonium salt or the like is added to soft polyvinyl chloride, since the fixed charge density is low and ion diffusion in the membrane is slow, this model can be applied.

On the other hand, diffusion gradually occurs in the membrane, and the concentration gradient changes in the order of x1, x2, x3, and x4 as indicated by arrows. When the interfering ions reach the internal solution, ion exchange occurs between the internal solution and the membrane. Since Cl ions are sufficiently present in the internal solution and diffusion is sufficiently faster than in the membrane, the ion composition of the boundary layer in the internal solution hardly changes. After a certain period of time, an equilibrium state is obtained. Note that it takes a long time to obtain the equilibrium state.

On the other hand, in a case where the ion exchange reaction is sufficiently faster than the ion diffusion in the boundary layer, the boundary layer diffusion rate-limiting mode 920 is obtained. In a case where the sample solution containing interfering ions comes in contact with the membrane, ions in the solution are exchanged for ions in the membrane. Since the ion exchange capacity of the membrane is great and interfering ions incorporated to the membrane surface diffuse in the membrane, the concentration of interfering ions on the membrane surface hardly increases.

On the other hand, since the ion diffusion in the boundary layer of the sample solution is slower than that in the ion exchange reaction, the supply of interfering ions from a bulk layer of the sample does not catch up. In the boundary layer of the sample solution near the membrane, the concentration of the interfering ions gradually decreases in the order of y1, y2, y3, and y4 as indicated by arrows. In this way, the composition ratio of the ions of the sample solution near the membrane surface changes until the equilibrium state is obtained. In the present embodiment, it is considered that a phenomenon occurs at a boundary layer diffusion rate-limiting mode indicated by the reference numeral 920 is captured. Note that FIG. 9 described above is an image diagram schematically illustrating the direction of the concentration change in each domain, and is considered not to be a linear profile in practice.

However, even at the boundary layer diffusion rate-limiting mode 920, when the ion exchange reaction of the membrane is too early with respect to the concentration of the interfering ions in the sample solution, the interfering ions in the boundary layer of the sample solution are instantaneously depleted. Thus, the change over time may be difficult to know depending on devices. On the contrary, when the ion exchange reaction of the membrane is too slow with respect to the concentration of the interfering ions in the sample solution, the change may be small and difficult to know. Therefore, the present embodiment can be easily applied by selecting a sensitive membrane having an appropriate ion exchange rate in accordance with the ion concentration region to be desirably detected and the time scale measured by the device.

Preferable conditions for using the principle of the present embodiment include (1) a device is capable of acquiring a potential in a solution stationary state immediately after introduction of a sample solution in an appropriate time domain, (2) the ion-sensitive membrane of the electrode has high-density fixed charges, (3) the internal solution contains ions to be measured at a high concentration, and (4) an operation for maintaining an ion balance in the membrane is periodically performed (for example, the solution containing the ions to be measured is periodically measured as illustrated in FIG. 3 by an operation for periodically flowing the solution containing main ions).

In the present embodiment, all of the above conditions (1) to (4) are satisfied. In an automatic analysis device that needs to measure a biological sample such as serum at a high throughput, an ion exchange membrane-based ion sensitive membrane is a more appropriate selection.

The standard of "high concentration" in the condition (3) can be appropriately determined by a person skilled in the art. For example, the standard of "high concentration" in the condition (3) may be an upper limit or a higher value of the concentration range of the ions to be measured that is usually assumed to be contained in the specimen. The standard of "high concentration" in the condition (3) may be an upper limit or a higher value of the concentration range of the ions to be measured that can be measured by the electrolyte concentration measurement device 100.

Since the ion exchange membrane has a high ion exchange capacity, it is sometimes referred to as a high-capacity ion-exchanger from an academic viewpoint. This high exchange capacity is a performance achieved by using a membrane having high-density fixed charges. On the contrary, for example, in the case of the Cl ion-sensitive membrane having a different membrane structure, such as a soft polyvinyl chloride membrane containing a general quaternary ammonium salt, the Cl ion-sensitive membrane does not have such high-density fixed charges. Accordingly, a person skilled in the art can clearly determine whether a membrane having high-density fixed charges is used as the sensitive membrane, based on the type of membrane or the like.

Here, a method for correcting a temperature of the potential waveform will be described. Even when a sample solution having a different temperature is introduced into the ion sensitive membrane and the solution is stationary, a temporal slope occurs in the potential waveform depending on the direction and degree of the temperature difference. This temperature difference affects not only the potential of the Cl ion electrode but also the potentials of the Na and K ion electrodes.

The direction of the potential change over time is reversed by the sign of the charges of the ions to be measured and the direction of the temperature difference, and the degree of influence thereof changes in accordance with the thickness of the sensitive membrane or the like. In the present embodiment, in a case where the temperature of the sample solution is lower than the temperature of the electrode, the potential of the Cl ion electrode at the time the solution is stationary has a positive slope over time, and the potentials of the Na and K ion electrodes have a negative slope. In a case where the sample solution temperature is higher than the electrode temperature, the opposite tendency is exhibited.

Since the membranes of the Na and K ion electrodes in the present embodiment are thicker than the membrane of the Cl ion electrode, the slope of the potential is greater than of the Cl ion electrode by a certain ratio. The storage unit 182 stores information about the correlation regarding the characteristics of the Na, K, and Cl ion electrodes regarding the change in potential due to the influence of such a temperature difference.

In S331 of FIG. 3, the interfering ion analysis unit 181 extracts the potential waveforms of the Na and K ion electrodes while the solution is stationary, the potential waveforms being measured by the potential measurement unit 171, as potential waveforms for temperature analysis. Based on the extracted potential waveforms, the degree of the influence of the temperature difference in the measurement (for example, slope in the potential change over time) is calculated (S332).

The temperature influence is corrected from the potential waveforms for interfering ion analysis using the correlation of the characteristic of each electrode with respect to the temperature difference stored in the storage unit 182 (S322). For example, different coefficients are stored for the respective ion-selective electrodes, and the coefficients are multiplied by the slope in the potential change over time. More specifically, since the change in potential is insensitive to temperature when a membrane is thin, but sensitive to temperature when a membrane is thick, coefficients according to the thickness of the membranes can be stored.

In this manner, the interfering ion analysis unit 181 compares the potential waveform of the ion-selective electrode that responds to ions to be measured with the potential waveform of the electrode that responds to ions having an opposite electric charge to an electric charge of the ions to be measured. The Na and K ion electrodes are not disturbed by anions and are not easily disturbed by cations. Therefore, it is possible to appropriately correct the temperature influence on the potential waveform of the Cl ion electrode by calculating the temperature influence based on the potential waveforms of the Na and K ion electrodes.

In the electrolyte analysis device for analyzing a biological sample as in the present embodiment, the characteristics of a specimen to be measured are often known in advance to some extent. Therefore, the accuracy of detecting the influence of interfering ions can be improved by inputting the information of the characteristics in advance.

For example, serum generally contains 30 to 40 mM of $HCO_3^-$. In a case where calibration is performed in a state where $HCO_3^-$ is contained for serum analysis at the time of calibration, the Cl concentration becomes low when control serum or the like not containing $HCO_3^-$ is analyzed in a conventional device. However, in the present embodiment, by inputting specimen information about serum in advance, in a case where a potential waveform does not have a negative slope, it can be detected that a specimen not containing $HCO_3^-$, such as a lyophilized sample of a control serum, might have been measured.

In the sequence of the present embodiment, the potential waveform is less likely to be obtained while the solution is stationary after the introduction of the sample solution. However, in a sequence where the sample is introduced after the reference electrode solution is introduced first, the potential waveform immediately after the introduction of the sample solution can be acquired with less disturbance, and thus the analysis becomes simpler.

The channel configuration, the structure of the reference electrode, and the like may be structures different from those of the present embodiment. The analysis method may be a method different from the method in the present embodiment. The magnitude and direction of the difference may be obtained based on the potentials at two or more points at different times while the solution is stationary.

Temperature correction is not always necessary, and does not have to be made based on the waveform of the internal standard solution. When the above conditions are satisfied, the influence of interfering ions can be similarly detected for an anion electrode and a cation electrode other than Cl ion electrode. When the influence of interfering ions is detected for the cation electrode, the above-described temperature correction method is preferably devised. On the other hand, a sensitive membrane having higher selectivity is less likely to be produced with an anion electrode as described above, the application to an anion electrode that is susceptible to interfering ions is more useful.

As described above, the electrolyte concentration measurement device according to the present embodiment can more easily determine the type or concentration of interfering ions. Further, such determination can be made with one electrode.

[Second embodiment]

An electrolyte concentration measurement device according to a second embodiment is different from that of the first embodiment in that the result of analyzing the influence of interfering ions is reflected in the calculation of the Cl ion concentration, and the Cl ion concentration for which the influence of interfering ions has been corrected is calculated. A device configuration and a calibration method are similar to those in the first embodiment.

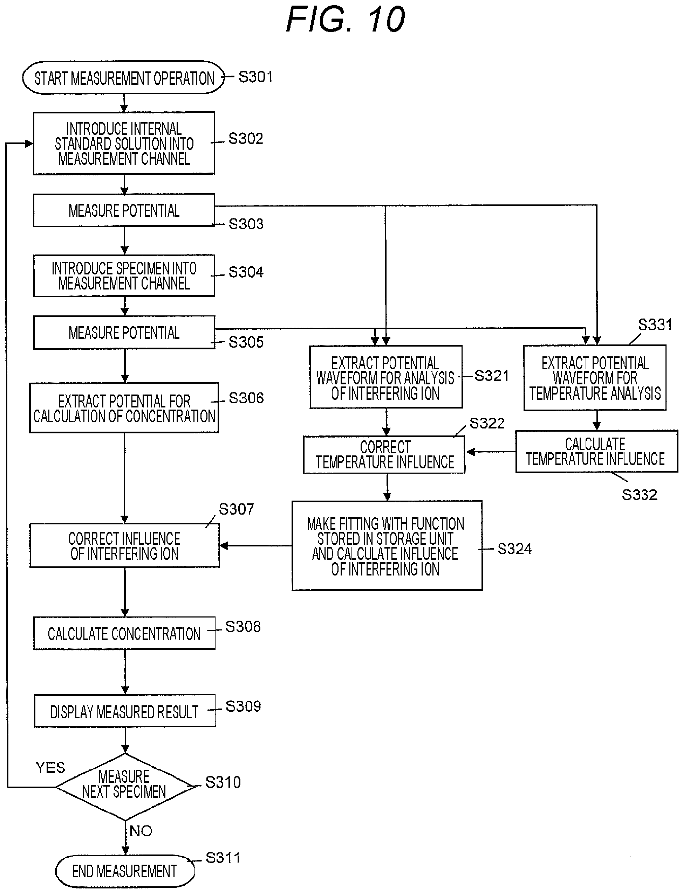

Here, a flow of continuous analysis in the device according to the present embodiment is illustrated in FIG. 10. A difference from the flow of the first embodiment is that the influence of interfering ions is calculated by fitting the function stored in the storage unit 182 to the obtained potential waveform (S324), and the potential for concentration calculation is corrected using the calculated result (S307). In this way, the Cl ion concentration can be calculated in a form where the influence of interfering ions is removed.

In the calculation of the present embodiment, S324, S307, and S308 are simultaneously executed. Specifically, the storage unit 182 stores, as a change model of a potential time for each interfering ion (j), a function $F_j(C_j, t)$ having a concentration ($C_j$) and a time ($t$) as variables. The potential waveform obtained in S306 is fitted to the potential waveform for interfering ion analysis so that the Cl ion concentration ($C_{Cl}$) and each interfering ion concentration ($C_j$) are obtained. The following equation (6) holds for expressing the above.

$$E(t) = G(C_{Cl}) + \Sigma_j[F_j(C_j, t)] \quad \ldots \text{Equation (6)}$$

E (t) represents a measured potential waveform. G ($C_{Cl}$) represents a potential value at a certain Cl ion concentration with a function having a concentration ($C_{Cl}$) as a variable. Since this does not change with time, the variable of the time t is not included. The analysis accuracy of these functions is improved by reflecting information, such as the slope sensitivity, selectivity, and ion exchange rate of the actually used electrodes. Accordingly, these pieces of information may be input into the storage unit 182 by the user or may be obtained in advance by measurement. The input unit 176 may be used to input the characteristics (e.g. $G(C_{Cl})$ and/or the function $F_j(C_j, t)$) of the ion-selective electrode to the storage unit 182.

In this manner, the concentration calculation unit 172 corrects the influence detected by the interfering ion analysis unit 181 and calculates the concentration of the ions to be measured.

Note that the Cl ion electrode has selectivity, partition coefficient, ion exchange reaction rate, and diffusion rate that are different depending on the species and concentrations of interfering ions in the sample solution. Thus, the analysis is conducted by using the fact that different potential waveforms exhibit. On the other hand, combinations of specific ion species and concentrations may have similar waveforms. This case makes it difficult to calculate the type and concentration of interfering ions.

However, in the electrolyte analysis device according to the present embodiment, the characteristics of a specimen to be measured are often known in advance to some extent. Therefore, the accuracy of calculating the concentrations of Cl ions and interfering ions can be improved by inputting the information in advance.

For example, serum generally contains 30 mM to 40 mM of $HCO_3^-$. In the present embodiment, when specimen information indicating that the specimen is serum is input in advance, the analysis such as fitting can be conducted preferentially for a function of $HCO_3^-$ because the potential waveform usually exhibits a negative slope.

Interfering ions such as $Br^-$, which are not usually contained in blood, may be contained in a specimen due to the inoculation of a drug or the like. Even in such a case, the medication information about a patient may be inputted as the information about the specimen to the device in advance. Thereby, the waveform analysis can be conducted with priority given to the fitting of the function of the interfering ion species according to the drug, and thus the analysis accuracy can be improved. The information about the specimen may indicate, for example, the type of a medication that may be contained in the specimen. The storage unit 182 may store the type of a medication and the type of interfering ions contained in the medication in association with each other.

In this way, since the type of the interfering ions is specified by inputting the type of the medication, the concentration of the interfering ions can be estimated more accurately. Further, not only the Cl ion concentration but also the concentration of interfering ions due to medication administration can be calculated, and there is also a possibility that the concentration of interfering ions may be used as an index of pharmacokinetics.

Note that the input unit 176 and the output unit 174 of the present embodiment can be used by a user to directly input information or directly view an output. The input unit 176 and the output unit 174 can also cooperate with other information systems, such as an electronic medical record and medication information system, and a device integrated monitoring system.

As described above, the electrolyte concentration measurement device according to the present embodiment can more easily determine the type or concentration of interfering ions as in the first embodiment. Further, such determination can be made with one electrode.

[Third Embodiment]

The configuration of an electrolyte concentration measurement device according to a third embodiment is different from that of the first embodiment in that an anion electrode having characteristics different from those of a Cl ion electrode is mounted in addition to Na, K, and Cl ion electrodes. The anion electrode to be added is different from other anion electrodes in some or all of ion selectivity, ion exchange reaction rate, ion diffusion coefficient in a sensitive membrane, fixed charge density, internal solution type, and main ion type. As described above, the electrolyte concentration measurement device according to the present embodiment has N ion-selective electrodes having different characteristics.

The number of electrodes to be additionally mounted varies depending on the number of ion species to be desirably analyzed. In the present embodiment, N anion electrodes include Cl ion electrodes. That is, when a certain sample solution is measured, N potential waveforms different depending on species and concentration of ions contained in the sample solution and electrode characteristics are obtained.

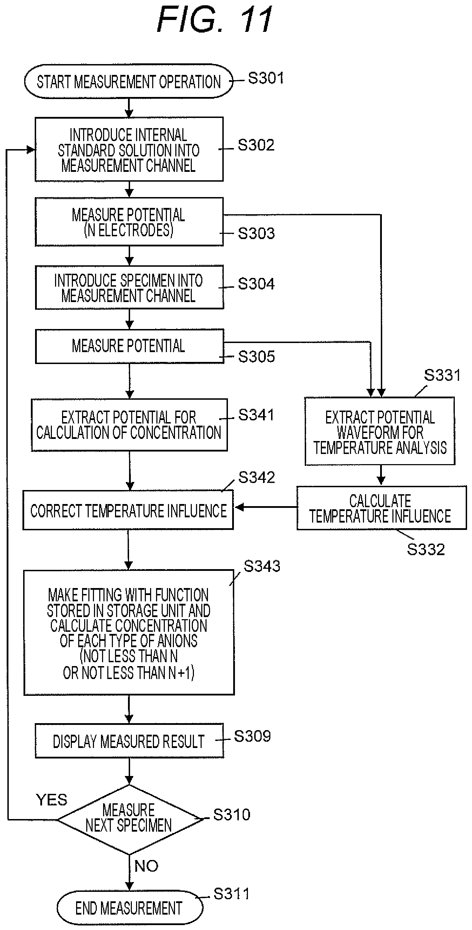

Here, a flow of continuous analysis in the present embodiment is illustrated in FIG. 11. Differences from FIG. 10 which illustrates the flow of the second embodiment will be mainly described.

The concentrations of the Na and K ions are calculated from the potentials at the stable timing as in FIG. 10. As for the anion concentration, N potential waveforms obtained from the N anion electrodes at a time the solution is stationary are extracted (S341). As in the second embodiment, the correction of the temperature influence on these waveforms is performed (S342).

Thereafter, the potential waveform of each electrode is fitted using the function regarding time as to the anion species and the concentration of each electrode stored in the storage unit. The type and concentration of the anions is calculated by integrating all the fitting results and analyzing the integrated result (S343).

This method makes it possible to obtain, from the potential waveforms of the N ion-selective electrodes, the concentrations of N or more types of ions. Further, the ion concentrations for N+1 or more ion species can also be measured based on time-series potential waveforms obtained respectively from the N ion-selective electrodes. A specific method for calculating the N+1 values based on the N waveforms can be appropriately designed by a person skilled in the art, based on a publicly-known technique. Note that the analysis method used in S343 is not particularly limited, and machine learning, a neural network, or the like may be used.

As described above, the electrolyte concentration measurement device according to the present embodiment can more easily determine the type or concentration of interfering ions as in the first and second embodiments.

The present invention is not limited to the above-described embodiments, and includes various modifications. For example, the above-described embodiments have been described in detail for easy understanding of the present invention, and are not necessarily limited to those having all the described configurations. Further, a part of the configuration of one embodiment can be replaced with the configuration of another embodiment, and the configuration of the other embodiment can be added to the configuration of one embodiment. In addition, the other configuration can be added to, deleted from a part of the configuration in each embodiment, and a part of the configuration in each embodiment can be replaced with the other configurations.

Reference Signs List

    100 electrolyte concentration measurement device (electrolyte analysis device)
    101 Cl ion electrode (ion-selective electrode)
    102 K ion electrode (ion-selective electrode)
    103 Na ion electrode (ion-selective electrode)
    104 reference electrode
    105 pinch valve
    106 vacuum aspiration nozzle
    107 sipper nozzle
    108 diluent supply nozzle
    109 internal standard solution supply nozzle
    110 dilution tank
    111 waste tank
    112 vacuum pump
    120 liquid junction
    121 to 127 solenoid valve
    131 internal standard solution syringe pump
    132 diluent syringe pump
    133 sipper syringe
    141 internal standard solution bottle
    151 diluent bottle
    161 reference electrode solution bottle
    170 measurement unit
    171 potential measurement unit
    172 concentration calculation unit
    174 output unit
    175 device control unit
    176 input unit
    181 interfering ion analysis unit
    182 storage unit
    611 sensitive membrane
    701 Cl ion electrode (ion-selective electrode)
    702 reference electrode
    711 sample solution syringe
    712 syringe
    910 intra-membrane diffusion rate-limiting mode
    920 boundary layer diffusion rate-limiting mode

**EP 4 502 590 A1**

All publications, patents, and patent applications cited herein are hereby incorporated by reference in their entirety.

**Claims**

1. An electrolyte analysis device having an ion-selective electrode and using potential measurement, the device comprising:

   a storage unit configured to store a relationship of a potential change over time with respect to an interfering ion; and
   an interfering ion analysis unit configured to detect an influence of the interfering ion based on the potential change over time obtained from the ion-selective electrode while a specimen solution is stationary after the specimen solution comes in contact with the ion-selective electrode.

2. The electrolyte analysis device according to claim 1, wherein the interfering ion analysis unit is configured to identify a type of the interfering ion based on a direction of a change in a potential waveform.

3. The electrolyte analysis device according to claim 1, wherein the interfering ion analysis unit is configured to calculate a concentration of the interfering ion based on a change slope in a potential waveform.

4. The electrolyte analysis device according to claim 1, further comprising:

   a concentration calculation unit configured to calculate a concentration of an ion to be measured,
   wherein the concentration calculation unit is configured to correct the influence and calculates the concentration of the ion to be measured, based on the influence detected by the interfering ion analysis unit.

5. The electrolyte analysis device according to claim 1, wherein an ion to be measured is an anion.

6. The electrolyte analysis device according to claim 1,
   wherein the interfering ion analysis unit is configured to compare a potential waveform of the ion-selective electrode with a potential waveform of an electrode that responds to an ion having an opposite electric charge to an electric charge of an ion to be measured.

7. The electrolyte analysis device according to claim 1, wherein the ion-selective electrode is a flow-type ion-selective electrode.

8. The electrolyte analysis device according to claim 1, wherein the ion-selective electrode has a membrane having high-density fixed charges as a sensitive membrane.

9. The electrolyte analysis device according to claim 1, wherein the ion-selective electrode has an ion exchange membrane as a sensitive membrane.

10. The electrolyte analysis device according to claim 1, wherein the ion-selective electrode has an internal solution, and the internal solution contains high concentration of ions to be measured.

11. The electrolyte analysis device according to claim 1, wherein the solution containing ions to be measured is periodically measured.

12. The electrolyte analysis device according to claim 1, further comprising an output unit configured to display a detection result of the influence of the interfering ion.

13. The electrolyte analysis device according to claim 1, further comprising an input unit that is used for inputting a characteristic of the ion-selective electrode in the storage unit.

14. The electrolyte analysis device according to claim 1, further comprising an input unit that is used for inputting information about the specimen in the storage unit.

**15.** The electrolyte analysis device according to claim 1,

wherein the ion-selective electrode includes N ion-selective electrodes having different characteristics, and wherein ion concentrations of N+1 or more ion species are measured based on time-series potential waveforms obtained respectively from the ion-selective electrodes.

# FIG. 1

# FIG. 2

```
┌─────────────────────────────┐
│       ACTIVATE DEVICE       │── S201
└─────────────────────────────┘
               │
               ▼
┌─────────────────────────────┐
│      PROVIDE ELECTRODE      │── S202
└─────────────────────────────┘
               │
               ▼
┌─────────────────────────────┐
│    PROVIDE REAGENT BOTTLE   │── S203
└─────────────────────────────┘
               │
               ▼
┌─────────────────────────────┐
│       REAGENT PRIMING       │── S204
└─────────────────────────────┘
               │
               ▼
┌─────────────────────────────┐
│      MEASURE INTERNAL       │── S205
│     STANDARD SOLUTION       │
└─────────────────────────────┘
               │
               ▼
┌─────────────────────────────┐
│      MEASURE STANDARD       │── S206
│    SOLUTION L AND H, AND    │
│    THEN CALCULATE SLOPE     │
└─────────────────────────────┘
               │
               ▼
┌─────────────────────────────┐
│         CALCULATE           │── S207
│  CONCENTRATION OF INTERNAL  │
│      STANDARD SOLUTION      │
└─────────────────────────────┘
```

# FIG. 3

START MEASUREMENT OPERATION — S301

↓

INTRODUCE INTERNAL STANDARD SOLUTION INTO MEASUREMENT CHANNEL — S302

↓

MEASURE POTENTIAL — S303

↓

INTRODUCE SPECIMEN INTO MEASUREMENT CHANNEL — S304

↓

MEASURE POTENTIAL — S305

↓

EXTRACT POTENTIAL FOR CALCULATION OF CONCENTRATION — S306

↓

CALCULATE CONCENTRATION — S308

↓

DISPLAY MEASURED RESULT — S309

↓

MEASURE NEXT SPECIMEN — S310

YES → (back to S302)

NO

↓

END MEASUREMENT — S311

EXAMPLE: PERIODICALLY MEASURE

EXTRACT POTENTIAL WAVEFORM FOR ANALYSIS OF INTERFERING ION — S321

↓

CORRECT TEMPERATURE INFLUENCE — S322

↓

CALCULATE INFLUENCE OF INTERFERING ION — S323

EXTRACT POTENTIAL WAVEFORM FOR TEMPERATURE ANALYSIS — S331

↓

CALCULATE TEMPERATURE INFLUENCE — S332

19

# FIG. 4

# FIG. 5

# FIG. 6

610

620

# FIG. 7

# FIG. 8

# FIG. 9

# FIG. 10

START MEASUREMENT OPERATION — S301

INTRODUCE INTERNAL STANDARD SOLUTION INTO MEASUREMENT CHANNEL — S302

MEASURE POTENTIAL — S303

INTRODUCE SPECIMEN INTO MEASUREMENT CHANNEL — S304

MEASURE POTENTIAL — S305

EXTRACT POTENTIAL FOR CALCULATION OF CONCENTRATION — S306

EXTRACT POTENTIAL WAVEFORM FOR ANALYSIS OF INTERFERING ION — S321

EXTRACT POTENTIAL WAVEFORM FOR TEMPERATURE ANALYSIS — S331

CORRECT TEMPERATURE INFLUENCE — S322

CALCULATE TEMPERATURE INFLUENCE — S332

MAKE FITTING WITH FUNCTION STORED IN STORAGE UNIT AND CALCULATE INFLUENCE OF INTERFERING ION — S324

CORRECT INFLUENCE OF INTERFERING ION — S307

CALCULATE CONCENTRATION — S308

DISPLAY MEASURED RESULT — S309

MEASURE NEXT SPECIMEN — S310

YES

NO

END MEASUREMENT — S311

# FIG. 11

START MEASUREMENT OPERATION — S301

INTRODUCE INTERNAL STANDARD SOLUTION INTO MEASUREMENT CHANNEL — S302

MEASURE POTENTIAL (N ELECTRODES) — S303

INTRODUCE SPECIMEN INTO MEASUREMENT CHANNEL — S304

MEASURE POTENTIAL — S305

EXTRACT POTENTIAL FOR CALCULATION OF CONCENTRATION — S341

EXTRACT POTENTIAL WAVEFORM FOR TEMPERATURE ANALYSIS — S331

CORRECT TEMPERATURE INFLUENCE — S342

CALCULATE TEMPERATURE INFLUENCE — S332

MAKE FITTING WITH FUNCTION STORED IN STORAGE UNIT AND CALCULATE CONCENTRATION OF EACH TYPE OF ANIONS (NOT LESS THAN N OR NOT LESS THAN N+1) — S343

DISPLAY MEASURED RESULT — S309

MEASURE NEXT SPECIMEN — S310

YES

NO

END MEASUREMENT — S311

## INTERNATIONAL SEARCH REPORT

| International application No. |
| --- |
| **PCT/JP2023/002294** |

**A.    CLASSIFICATION OF SUBJECT MATTER**

*G01N 27/416*(2006.01)i; *G01N 27/26*(2006.01)i; *G01N 27/28*(2006.01)i
FI:    G01N27/416 351K; G01N27/26 371D; G01N27/26 371F; G01N27/28 321; G01N27/416 351B

According to International Patent Classification (IPC) or to both national classification and IPC

**B.    FIELDS SEARCHED**

Minimum documentation searched (classification system followed by classification symbols)

G01N27/416; G01N27/26; G01N27/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); Scopus

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2020-12823 A (CANON MEDICAL SYSTEMS CORP) 23 January 2020 (2020-01-23) paragraphs [0003], [0006], [0013]-[0014], [0035]-[0036], [0040], [0049], [0071]-[0072], [0074], [0097]-[0099], [0101]-[0103], [0116], [0159], fig. 1-4, 10-11 | 1, 3-5, 7-10, 12-14 |
| A | entire text, all drawings | 2, 6, 11, 15 |
| A | JP 2003-501656 A (BROADLEY TECHNOLOGIES CORPORATION) 14 January 2003 (2003-01-14) paragraph [0069] | 1-15 |
| A | US 2017/0276630 A1 (PARKER-HANNIFIN CORPORATION) 28 September 2017 (2017-09-28) paragraph [0012] | 1-15 |
| A | WO 2009/084675 A1 (HORIBA, LTD.) 09 July 2009 (2009-07-09) paragraph [0025] | 1-15 |
| A | JP 2020-41968 A (HITACHI HIGH-TECHNOLOGIES CORPORATION) 19 March 2020 (2020-03-19) paragraph [0031] | 1-15 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 March 2023** | **14 March 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/002294**

| C. | DOCUMENTS CONSIDERED TO BE RELEVANT | | |
|---|---|---|
| Category* | Citation of document, with indication, where appropriate, of the relevant passages | Relevant to claim No. |
| A | MORF, W. E. Dynamic Response Behavior of Ion-Selective Electrodes. THE PRINCIPLES OF ION-SELECTIVE ELECTRODES AND OF MEMBRANE TRANSPORT. Chapter 14. Akademiai Kiado. Budapest. 1981, pp. 375-400, ISBN 963 05 2511 9<br>pp. 392-397, fig. 14.5 | 1-15 |

Form PCT/ISA/210 (second sheet) (January 2015)

International application No.

**PCT/JP2023/002294**

| Patent document cited in search report | | | Publication date (day/month/year) | Patent family member(s) | | | Publication date (day/month/year) |
|---|---|---|---|---|---|---|---|
| JP | 2020-12823 | A | 23 January 2020 | CN | 110702767 | A | |
| JP | 2003-501656 | A | 14 January 2003 | US paragraph [0095] WO | 2001/0045357 2000/075648 | A1 A1 | |
| US | 2017/0276630 | A1 | 28 September 2017 | US | 2021/0356418 | A1 | |
| WO | 2009/084675 | A1 | 09 July 2009 | EP paragraph [0025] US CN | 2228644 2010/0259271 101855543 | A1 A1 A | |
| JP | 2020-41968 | A | 19 March 2020 | US paragraph [0039] WO EP CN | 2021/0318266 2020/054473 3851842 112654862 | A1 A1 A1 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000121595 A
- US 20130304395 A
- WO 2019163281 A
- JP 2022049361 A